# EUROPEAN PATENT SPECIFICATION

(11) **EP 4 337 088 B1**
(45) Date of publication and mention of the grant of the patent: **24.06.2026**
(21) Application number: 22808126.1
(22) Date of filing: 09.05.2022
(51) Int. Cl.: A61B 5/0205, A61B 5/0245, A61B 5/05, A61B 5/08

(54) **SYSTEM AND METHOD FOR QUANTIFICATION OF RESPIRATION**
SYSTEM UND VERFAHREN ZUR QUANTIFIZIERUNG DER ATMUNG
SYSTÈME ET PROCÉDÉ DE QUANTIFICATION DE LA RESPIRATION

(30) Priority: 10.05.2021 US 202163186318 P
(43) Date of publication of application: 20.03.2024
(73) Proprietor: Vanderbilt University, Nashville, TN 37240 (US)
(72) Inventor: HOCKING, Kyle M., Nashville, Tennessee 37240 (US); BROPHY, Colleen M., Nashville, Tennessee 37240 (US); ALVIS, Bret D., Nashville, Tennessee 37240 (US)
(74) Representative: Kehl, Ascherl, Liebhoff & Ettmayr Patentanwälte Partnerschaft mbB
(86) International application number: PCT/US2022/028332
(87) International publication number: WO 2022/240747

(56) References cited:
- WO-A1-2018/112354
- WO-A1-2019/217778
- US-A- 4 862 361
- US-A1- 2006 079 773
- US-A1- 2015 173 689
- US-A1- 2016 151 628
- US-A1- 2017 360 374
- US-A1- 2019 110 745
- SCHOLKMANN FELIX ET AL: "The Pulse-Respiration Quotient: A Powerful but Untapped Parameter for Modern Studies About Human Physiology and Pathophysiology", FRONTIERS IN PHYSIOLOGY, vol. 10, 9 April 2019 (2019-04-09), CH, XP093264912, ISSN: 1664-042X, DOI: 10.3389/fphys.2019.00371

## Description

### CROSS REFERENCE TO RELATED APPLICATION

The present application is derived from an international application claiming priority to U.S. provisional application no. 63/186,318, filed May 10, 2021.

The following document is also cited: Respiratory Non-Invasive Venous Waveform Analysis for Assessment of Respiratory Distress in Coronavirus Disease 2019 Patients: An Observational Study, Alvis, Bret MD; Vaughn, Lexie MD; Schmeckpeper, Jeffrey MD, PhD; Huston, Jessica MD; Case, Marisa RN; Semler, Matthew MD; Lindenfeld, Jo Ann MD; Brophy, Colleen MD; Hocking, Kyle PhD, doi: 10.1097/CCE.0000000000000539.

### FEDERAL FUNDING STATEMENT

This invention was made with US government support under Grant No. BA- R01HL 148244-01 and Grant No. KH-R44HL 140669-01, awarded by the National Institutes of Health. The US government has certain rights in the invention.

### BACKGROUND

The novel coronavirus (SARS-CoV -2) has produced a global pandemic that continues to affect both economies and healthcare systems. Although most patients with SARS-CoV-2 experience only mild symptoms, a subset of infected patients will experience life-threatening respiratory failure. Evaluation and management of respiratory distress due to COVID-19 and other causes depends on the severity of the disease. Unfortunately, knowing whose symptoms will remain mild and who will progress to severe respiratory failure is difficult, and often requires observation in a hospital setting. Rapid and effective triage can be critical for early treatment and effective allocation of hospital resources.

WO 2018/112354 A1 describes a system and method for monitoring and determining patient parameters from sensed venous waveform, according to which a peripheral venous pressure is measured and used to detect levels, changes, or problems relating to patient blood volume. The peripheral venous pressure measurement is transformed from the time domain to the frequency domain for analysis. A heart rate frequency is identified, and harmonics of the heart rate frequency are detected and evaluated to determine, among other things, hypovolemia or hypervolemia, systemic vascular resistance, and cardiac and respiratory rates, and patient respiratory volume or effort.

WO 2019/217778 A1 describes a non-invasive venous waveform analysis for evaluating a subject, according to which a method embodiment includes generating, via a sensor of a computing device, a signal representing vibrations originating from a blood vessel of a subject and decomposing the signal into one or more first intrinsic oscillatory modes and one or more second intrinsic oscillatory modes. The one or more first intrinsic oscillatory modes have respective oscillation frequencies that are less than respective oscillation frequencies of the one or more second intrinsic oscillatory modes. The method includes obtaining an intensity spectrum of the one or more first intrinsic oscillatory modes over a range of frequencies and using the obtained intensity spectrum to determine a blood volume status of the subject. Another method embodiment includes using the one or more second intrinsic oscillatory modes to determine one or more mechanical properties of the blood vessel or tissue adjacent to the blood vessel.

### SUMMARY

The invention is defined in the appended claims. A first aspect of the disclosure is a system comprising: one or more processors; a user interface; a sensor; and a computer readable medium storing instructions that, when executed by the one or more processors, cause the system to perform functions comprising: generating, via the sensor, a signal representing vibrations originating from a blood vessel of a patient; generating an intensity spectrum of the signal that indicates intensities of the vibrations with respect to oscillation frequencies of the vibrations; identifying a first peak of the intensity spectrum that corresponds to a respiratory frequency of the patient and a second peak of the intensity spectrum that corresponds to a heart rate of the patient; performing a comparison of a first intensity of the first peak with a second intensity of the second peak; and generating, via the user interface, output indicative of the comparison.

A second aspect of the disclosure is a method comprising: generating, via the sensor, a signal representing vibrations originating from a blood vessel of a patient; generating an intensity spectrum of the signal that indicates intensities of the vibrations with respect to oscillation frequencies of the vibrations; identifying a first peak of the intensity spectrum that corresponds to a respiratory frequency of the patient and a second peak of the intensity spectrum that corresponds to a heart rate of the patient; performing a comparison of a first intensity of the first peak with a second intensity of the second peak; and generating, via the user interface, output indicative of the comparison.

A third aspect of the disclosure is a non-transitory computer readable medium storing instructions that, when executed by the system, cause the system to perform functions comprising: generating, via the sensor, a signal representing vibrations originating from a blood vessel of a patient; generating an intensity spectrum of the signal that indicates intensities of the vibrations with respect to oscillation frequencies of the vibrations; identifying a first peak of the intensity spectrum that corresponds to a respiratory frequency of the patient and a second peak of the intensity spectrum that corresponds to a heart rate of the patient; performing a comparison of a first intensity of the first peak with a second intensity of the second peak; and generating, via the user interface, output indicative of the comparison.

By the term "about" or "substantially" with reference to amounts or measurement values described herein, it is meant that the recited characteristic, parameter, or value need not be achieved exactly, but that deviations or variations, including for example, tolerances, measurement error, measurement accuracy limitations and other factors known to those of skill in the art, may occur in amounts that do not preclude the effect the characteristic was intended to provide.

The features, functions, and advantages that have been discussed can be achieved independently in various examples or may be combined in yet other examples further details of which can be seen with reference to the following description and drawings.

### BRIEF DESCRIPTION OF THE DRAWINGS

Figure 1 is a block diagram of a system, according to an example.
Figure 2 is a front view of a system, according to an example.
Figure 3 is a block diagram of a method, according to an example.
Figure 4 shows intensity spectra of vibrations originating from a patient's blood vessel, according to an example.
Figure 5 shows relationships between a calculated metric and hospitalization status and COVID-19 status, according to an example.
Figure 6 shows relationships of respiratory rate and oxygen saturation with hospitalization status and COVID-19 status, according to an example.
Figure 7 shows relationships of a calculated metric with lung disease, according to an example.
Figure 8 shows the predictive ability of a calculated metric, according to an example.

### DETAILED DESCRIPTION

The present disclosure relates generally to a system used (e.g., by a physician, a nurse, or another user) to determine the level of respiratory strain and/or distress that is occurring in a patient through measurement of the peripheral venous waveform via an external sensor. This measurement system and method are applicable to respiratory conditions and diseases that are both chronic and acute, including but not limited to: viral pneumonia, bacterial pneumonia, waning from mechanical ventilation, diseases requiring oxygen support therapy, sleep apnea, asthma, COPD, restrictive pulmonary diseases, obstructive pulmonary diseases, pneumonia, allergens, medicinal overdoses/side-effects, airway obstruction, pulmonary edema, cerebral vascular accidents, spinal cord injuries, opioid overdose/misuse, inhalational injuries, Guillain-barre syndrome, myasthenia gravis, amyotrophic lateral sclerosis (ALS), cystic fibrosis, trauma, sepsis, fibrotic/restrictive respiratory diseases, and/or autonomic function related diseases.

Non-Invasive Venous waveform Analysis (NIVA) is a promising monitoring approach for assessing vascular volume states in both adults and children. NIVA typically includes the use of a piezoelectric sensor on the volar aspect of the wrist to capture a venous waveform signal. The signal is then deconvoluted into two components with a fast Fourier Transformation into the frequency domain. The cardiac component (f0) and harmonics of the cardiac component are used to derive a value that is representative of the pulmonary capillary wedge pressure (PCWP), the most common data point for volume assessment.

Signals were collected from COVID-19 patients to assess volume status and the need for invasive measurements. Post hoc analysis was used to analyze and evaluate the respiratory component (fR0) of the venous waveform and the relationship of the respiratory component to the need for oxygen support therapy. Use of the respiratory component of the venous waveform can provide a rapid, non-invasive, inexpensive test to identify patients with COVID-19 at risk for requiring oxygen therapy.

The disclosure will now be described more fully hereinafter with reference to the accompanying drawings, in which exemplary embodiments of the disclosure are shown. This disclosure may, however, be embodied in many different forms and should not be construed as limited to the embodiments set forth herein. Rather, these embodiments are provided so that this disclosure will be thorough and complete, and will fully convey the scope of the disclosure to those skilled in the art. Like reference numerals refer to like elements throughout.

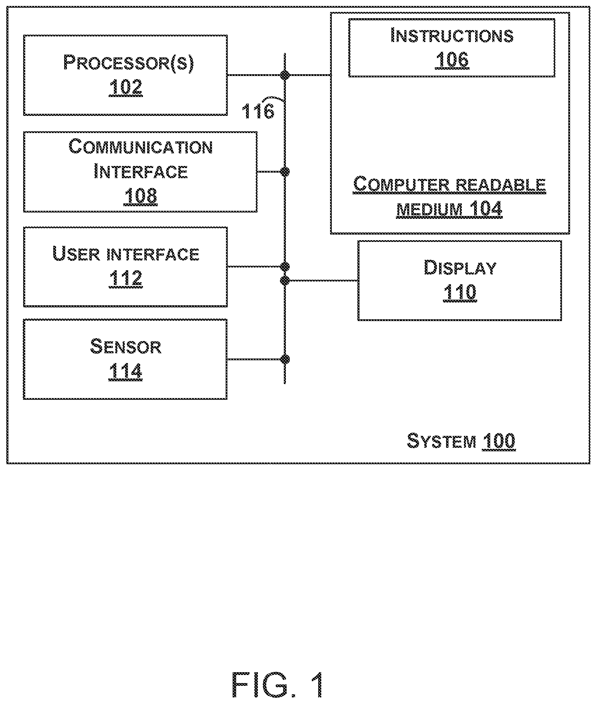

Figure 1 is a block diagram of a system 100. The system 100 includes one or more processors 102, a non-transitory computer readable medium 104 storing instructions 106, a communication interface 108, a display 110, a user interface 112, and a sensor 114. Components of the system 100 are linked together by a system bus, network, or other connection mechanism 116.

The one or more processors 102 can be any type of processor(s), such as a microprocessor, a digital signal processor, a multicore processor, etc., coupled to the non-transitory computer readable medium 104.

The non-transitory computer readable medium 104 can be any type of memory, such as volatile memory like random access memory (RAM), dynamic random access memory (DRAM), static random access memory (SRAM), or non-volatile memory like read-only memory (ROM), flash memory, magnetic or optical disks, or compact-disc read-only memory (CD-ROM), among other devices used to store data or programs on a temporary or permanent basis.

Additionally, the non-transitory computer readable medium 104 stores instructions 106. The instructions 106 are executable by the one or more processors 102 to cause the system 100 to perform any of the functions or methods described herein.

The communication interface 108 includes hardware to enable communication within the system 100 and/or between the system 100 and one or more other devices. The hardware can include transmitters, receivers, and antennas, for example. The communication interface 108 can be configured to facilitate communication with one or more other devices, in accordance with one or more wired or wireless communication protocols. For example, the communication interface 108 can be configured to facilitate wireless data communication for the system 100 according to one or more wireless communication standards, such as one or more Institute of Electrical and Electronics Engineers (IEEE) 801.11 standards, ZigBee standards, Bluetooth standards, etc. As another example, the communication interface 108 can be configured to facilitate wired data communication with one or more other devices.

The display 110 can be any type of display component configured to display data. As one example, the display 110 can include a touchscreen display. As another example, the display 110 can include a flat-panel display, such as a liquid-crystal display (LCD) or a light-emitting diode (LED) display.

The user interface 112 can include one or more pieces of hardware used to provide data and control signals to the system 100. For instance, the user interface 112 can include a mouse or a pointing device, a keyboard or a keypad, a microphone, a touchpad, or a touchscreen, among other possible types of user input devices. Generally, the user interface 112 can enable an operator to interact with a graphical user interface (GUI) provided by the system 100 (e.g., displayed by the display 110).

The sensor 114 generally takes the form of a piezioelectric sensor, an optical sensor, a multiplexed optical array, a pressure sensor, a force resistive sensor, a tonometer, an ultrasound sensor, a capacitive sensor, or a pressure transducer. For example, the sensor 114 can be a piezioelectric sensor that communicates wirelessly or via a wired connection with the one or more processors 102. Additionally or alternatively, the sensor 114 is securable to a skin of a patient via a strap and/or encapsulated within rubber, a polymer, polyurea, and/or silicone.

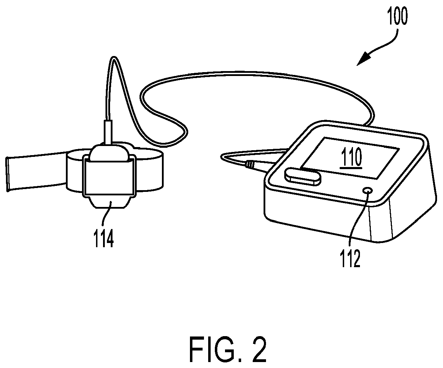

Figure 2 is a front view of the system 100. As shown, the system 100 includes the sensor 114 that communicates via a wired connection with the one or more processors 102 (not shown). Also shown in Figure 2 are the user interface 112 and the display 110.

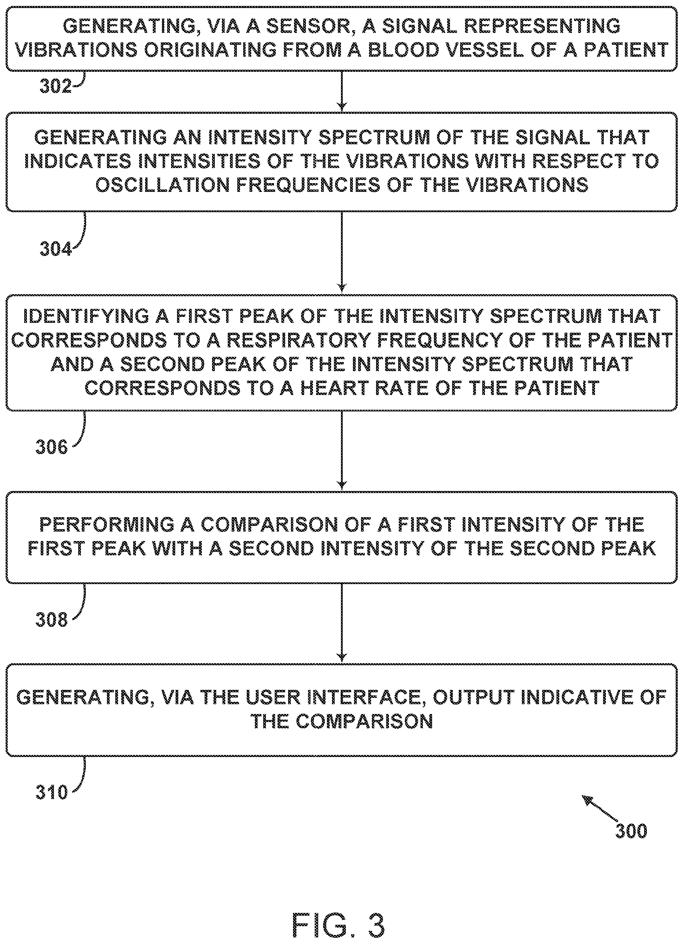

Figure 3 is a block diagram of a method 300. As shown in Figure 3, the method 300 includes one or more operations, functions, or actions as illustrated by blocks 302, 304, 306, 308, and 310. Although the blocks are illustrated in a sequential order, these blocks may also be performed in parallel, and/or in a different order than those described herein. Also, the various blocks may be combined into fewer blocks, divided into additional blocks, and/or removed based upon the desired implementation.

At block 302, the method 300 includes the system 100 generating, via the sensor 114, a signal representing vibrations originating from a blood vessel of a patient. In various examples, the blood vessel is a peripheral vein or a peripheral artery. The vibrations generally include vibrations generated by blood flowing against walls of the blood vessel or vibrations that are inherently present when a fluid like blood is flowing through a conduit like a blood vessel. Pulsatile flow is a result of forward moving blood from the arterial side as well as blood being pulled by the right side of the heart through the venous system. When the sensor 114 is a piezoelectric sensor, for example, the signal can represent displacement of the sensor 114 caused by the vibrations with respect to time. The signal generated by the sensor 114 can represent the vibrations that are generated via retrograde transmission of a negative pressure exerted on the patient's venous system by inspiratory pressures.

Typically, the sensor 114 is pressed against the patient's skin (e.g., with a back pressure of 10 mmHg to 60 mmHg) with a strap, microneedles, suction negative pressure or another restraint or attachment device while the system 100 generates the signal. In various examples, the sensor 114 is pressed against the skin at the wrist, the ankle, the eye, or the neck. In other examples, the sensor 114 is inserted into the ear canal.

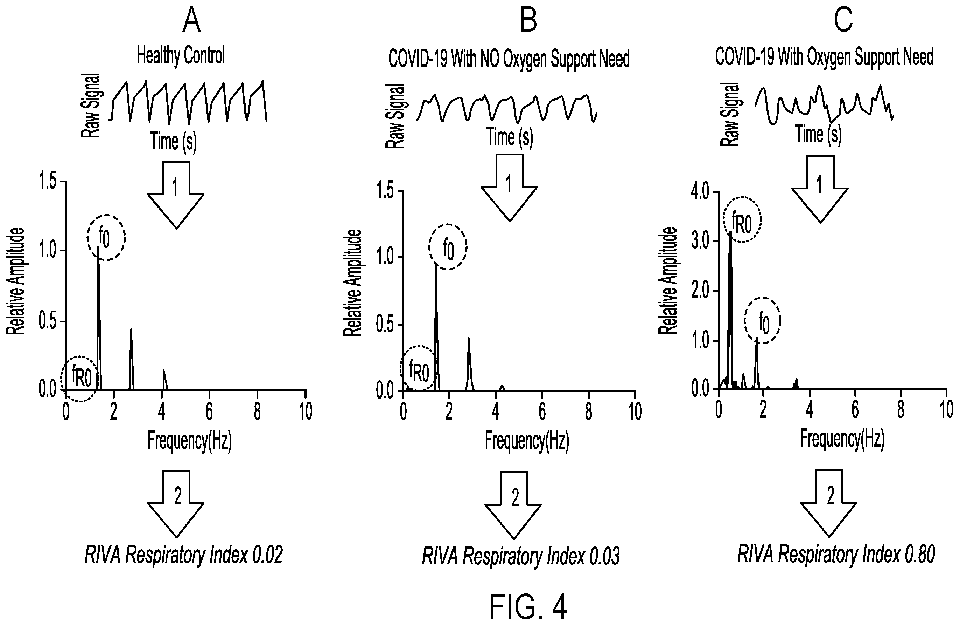

At block 304, the method 300 includes the system 100 generating an intensity spectrum of the signal that indicates intensities of the vibrations with respect to oscillation frequencies of the vibrations. The system 100 generally generates the intensity spectrum by performing a Fourier transform (e.g., a fast Fourier transform (FFT)) upon the signal. Figure 4 shows three examples of an intensity spectrum.

Figure 4 shows representative signals from patients with low risk to high risk of O₂ support need (left to right). Raw signals are transformed from the time domain (top) to the frequency domain (bottom). The relative amplitude of the respiratory rate (f_{R0}, fundamental frequency) compared to the relative amplitude of the pulse rate (f₀) is used to calculate a RIV A Respiratory Index (RIVA-RI). RIVA-RI is calculated based on the ratio of the relative amplitude of f_{R0} and f₀: RIVA-RI=f_{R0}/f₀

Panel A of Figure 4 represents a healthy control patient. Three easily recognizable peaks are shown at approximately 1.4 Hz (f₀), 2.8 Hz, and 4.2 Hz. The peak at 1.4 Hz (f₀) is the fundamental frequency or first harmonic of the patient's heartbeat, the peak at 2.8 Hz is the second harmonic of the patient's heartbeat, and the peak at 4.2 Hz is the third harmonic of the patient's heartbeat. There is a small peak at about 0.5 Hz that is the fundamental frequency or first harmonic of the patient's breathing rate (f_{R0}).

Panel B of Figure 4 represents a COVID positive patient with no need for supplemental oxygen treatment. Three easily recognizable peaks are shown at approximately at 1.4 Hz (f₀), 2.8 Hz, and 4.2 Hz. The peak at 1.4 Hz (f₀) is the fundamental frequency or first harmonic of the patient's heartbeat, the peak at 2.8 Hz is the second harmonic of the patient's heartbeat, and the peak at 4.2 Hz is the third harmonic of the patient's heartbeat. There is a small peak at about 0.5 Hz that is the fundamental frequency or first harmonic of the patient's breathing rate (f_{R0}).

Panel C of Figure 4 represents a COVID positive patient with a need for supplemental oxygen treatment. Two easily recognizable heartbeat peaks are shown at approximately at 1.8 Hz (f₀) and 3.6 Hz. The peak at 1.8 Hz (f₀) is the fundamental frequency or first harmonic of the patient's heartbeat and the peak at 3.6 Hz is the second harmonic of the patient's heartbeat. There is a large peak at about 0.5 Hz that is the fundamental frequency or first harmonic of the patient's breathing rate (f_{R0}).

At block 306, the method 300 includes the system 100 identifying a first peak of the intensity spectrum that corresponds to a respiratory frequency of the patient and a second peak of the intensity spectrum that corresponds to a heart rate of the patient.

Referring to panel A, panel B, and panel C of Figure 4, the system 100 identifies the first peak that corresponds to the respiratory frequency (f_{R0}) (*e.g.*, the fundamental respiratory frequency) of the patient and the second peak that corresponds to a heart rate (f₀) (*e.g.*, the fundamental heart beat frequency) of the patient. The system 100 identifies the first peak and the second peak automatically or manually.

For example, the system 100 can automatically identify the first peak (f_{R0}) as being a most intense peak of any peaks within a range of 0.1 Hz to 0.5 Hz, which is a range within which the fundamental respiratory frequency would be expected to be. In some cases, the patient's respiratory rate can be somewhat irregular, leading to a broader but less intense peak that corresponds to a range of frequencies instead of a singular frequency. As such, the system 100 can identify the first peak (f_{R0}) by determining that the first peak (f_{R0}) represents a greatest amount of energy (*e.g*., area under the curve of the intensity spectrum) of any peaks within the range of 0.1 Hz to 0.5 Hz.

Likewise, the system 100 can automatically identify the second peak (f₀) as being a most intense peak of any peaks within a range of 0.5 Hz to 3.5 Hz, which is a range within which the fundamental heart beat frequency would be expected to be. In some cases, the patient's heart rate can be somewhat irregular, leading to a broader but less intense peak that corresponds to a range of frequencies instead of a singular frequency. As such, the system 100 can identify the second peak (f₀) by determining that the second peak (f₀) represents a greatest amount of energy (*e.g*., area under the curve of the intensity spectrum) of any peaks within the range of 0.5 Hz to 3.5 Hz.

In some examples, the system 100 receives, via the user interface 112, an input explicitly identifying the first peak (f_{R0}) as corresponding to the fundamental respiratory frequency. More specifically, the user could identify the first peak (f_{R0}) using a touch screen gesture. In this context, the system 100 identifies the first peak (f_{R0}) based on the input received via the user interface 112.

Likewise, the system 100 receives, via the user interface 112, an input explicitly identifying the second peak (f₀) as corresponding to the fundamental heart beat frequency. More specifically, the user could identify the second peak (f₀) using a touch screen gesture. In this context, the system 100 identifies the second peak (f₀) based on the input received via the user interface 112.

In yet other examples, the system 100 receives an input from an additional sensor such as a pneumograph, with the input identifying the first peak (f_{R0}). As such, the system 100 identifies the first peak (f_{R0}) based on the received input. That is, the input received from the additional sensor explicitly indicates the respiratory rate and the system 100 identifies the first peak (f_{R0}) based on the respiratory rate indicated by the received input.

Likewise, the system 100 receives an input from an additional sensor such as a pulse sensor, with the input identifying the second peak (f₀). As such, the system 100 identifies the second peak (f₀) based on the received input. That is, the input received from the additional sensor explicitly indicates the heart rate and the system 100 identifies the second peak (f₀) based on the heart rate indicated by the received input.

At block 308, the method 300 includes the system 100 performing a comparison of a first intensity of the first peak with a second intensity of the second peak. For example, the system 100 calculates a ratio of the first intensity of the first peak (f_{R0}) to the second intensity of the second peak (f₀) or a ratio of the second intensity of the second peak (f₀) to the first intensity of the first peak (f_{R0}).

The greater the ratio of the first intensity of the first peak (f_{R0}) to the second intensity of the second peak (f₀), the more likely it is that the patient needs or will need supplemental oxygen therapy.

At block 310, the method 300 includes the system 100 generating, via the user interface 112, output indicative of the comparison (*e.g*., the ratio of the first intensity of the first peak (f_{R0}) to the second intensity of the second peak (f₀) or the ratio of the second intensity of the second peak (f₀) to the first intensity of the first peak (f_{R0})).

In various examples, the system 100 uses a table or an equation that maps values of the ratio to values of various health (*e.g*., respiratory) metrics. For example, data could be collected experimentally, with the data indicating how various health metrics correlate with the ratio discussed above. Statistical techniques can be used to define mathematical functions that define the relationship between each of the health metrics and the calculated ratio. As such, the system 100 can produce output that indicates the values of various metrics based on the calculated ratio.

For example, the output can indicate one or more of: a diffusing capacity of the patient's lung for carbon monoxide (DLCO), a diffusing capacity of the patient's lung for carbon monoxide divided by an alveolar volume (DLCO/VA), an inspiratory reserve volume (IRV) for the patient's lung, a maximum rate of oxygen consumption (VO₂ max), a work of breathing, a pleural pressure, or an inspiratory capacity (IC) for the patient's lung. As such, prior to generating the output, the system can determine, based on the comparison of the first intensity corresponding to the respiratory frequency and the second intensity corresponding to the heart rate, one or more of the DLCO, DLCO/VA, IRV, the VO₂ max, the work of breathing, the pleural pressure, or the IC, and generate the output accordingly.

In spontaneous breathing, the components of the work of breathing include: 1) work needed by the muscles of respiration to overcome elastic recoil of the lung and to displace the chest wall and abdomen, 2) the work needed to overcome airway resistance and lung viscosity, and 3) work needed to overcome inertia. Increased work of breathing is believed to be correlated with an increased ratio of the first intensity corresponding to the respiratory rate over the second intensity corresponding to the heart rate.

Treatment or therapy for the patient can be initiated, adjusted, or ceased based on the results of the comparison, *i.e.*, the outputs generated by the system 100. For example, a dosage, frequency, or duration of a supplemental oxygen treatment and/or a continuous positive airway pressure (CPAP) treatment for the patient can be altered based on the output of the system 100.

In various examples, the patient is experiencing a pulmonary disease such as chronic obstructive pulmonary disease (COPD), sleep apnea, bronchiectasis, asthma, pulmonary edema, congestion, pneumothorax, pneumonia, allergens, medicinal overdoses/side-effects, airway obstruction, pulmonary edema, cerebral vascular accidents, spinal cord injuries, opioid overdose/misuse, inhalational injuries, Guillain-barre syndrome, myasthenia gravis, amyotrophic lateral sclerosis (ALS), cystic fibrosis, sepsis, respiratory distress, acute respiratory distress syndrome, idiopathic pulmonary fibrosis, or pulmonary fibrosis, and the corresponding treatment can be informed by the results of the comparison

The ratio (RIVA-RI) of the first intensity corresponding to the respiratory rate over the second intensity corresponding to the heart rate being greater than 0.6 yielded a 92% sensitivity (95% CI, 61.52% to 99.79%) and 47% specificity (95% CI, 28.34% to 65.67%) in predicting a COVID-19+ patient's future need for oxygen support therapy.

Healthy control patients displayed significantly lower RIVA-RI values compared to patients with idiopathic pulmonary fibrosis.

When each adult (N=4, Ages= 38 years, 34 years, 22 years, and 21 years) reached their VO₂ max, RIVA-RI increased from a median baseline of 0.03 to a value of 0.78 suggesting that RIVA-RI increases relative to increasing oxygen demand.

Data collected from patients experiencing respiratory distress, defined by the need for oxygen support therapy, demonstrated a significant elevation in the respiratory venous waveform amplitude in relation to the cardiac venous waveform amplitude, that is, an increase in RIVA-RI.

Data from the preliminary observational study suggests that a RIVA-RI value of greater than or equal to 0.6 had an AUC=0.64 with a sensitivity of 92% and specificity of 47% in predicting a COVID-19+ patients' future need for oxygen support therapy.

### ADDITIONAL EXAMPLES

The coronavirus 2 (SARS-CoV-2) has led to a global pandemic that has crippled both economies and healthcare. The coronavirus pandemic highlighted the lack of innovation in respiratory monitoring. There is currently no non-invasive monitor available to obtain respiratory physiologic information except traditional spirometry, pulse oximetry, and various ways of counting respiration rates. These technologies have been around for decades. This pandemic proved more is necessary to adequately monitor and triage patients experiencing acute respiratory distress. One of the major issues with respiratory disease is it can quickly propagate to life-threatening respiratory failure. Whether it by COVID-19, traditional viral/bacterial pneumonia, asthma, and/or any other acute on chronic pulmonary disease, patients with mild symptoms typically can recover from home and not further burden the healthcare system. Unfortunately, knowing whose symptoms will remain mild and who will acutely progress to severe respiratory failure is difficult and, often requires observation in a hospital setting. The sudden deterioration of respiratory insufficiency to failure remains a major concern and often results in liberal hospital triage. The unique characteristics of respiratory disease and the vast caseload that can exist in a viral pandemic that primarily effects the pulmonary system, suggest that rapid and effective triage are critical for early treatment and effective allocation of hospital resources.

Peripheral venous waveforms can be captured non-invasively at the wrist using a piezo-electric sensor. These are low amplitude waveforms that typically require signal processing and amplification for analysis. The Fourier transformation can then be used to bundle windows of waveforms and present the data in the frequency (instead of time) domain. This leads to a low amplitude waveform at approximately 0.2 Hz (termed "f_{R0}") that corresponds to respiration (equivalent to 12 breaths/minute). This wave is likely due to retrograde propagation of the negative intrathoracic pressure during inspiration from the right atrium/vena cava, throughout the venous system. There is an additional peak waveform at about 1 Hz equivalent to the cardiac frequency. This signal and harmonics of the fundamental cardiac frequency have been used to develop an algorithm that allows for the evaluation of volume status.

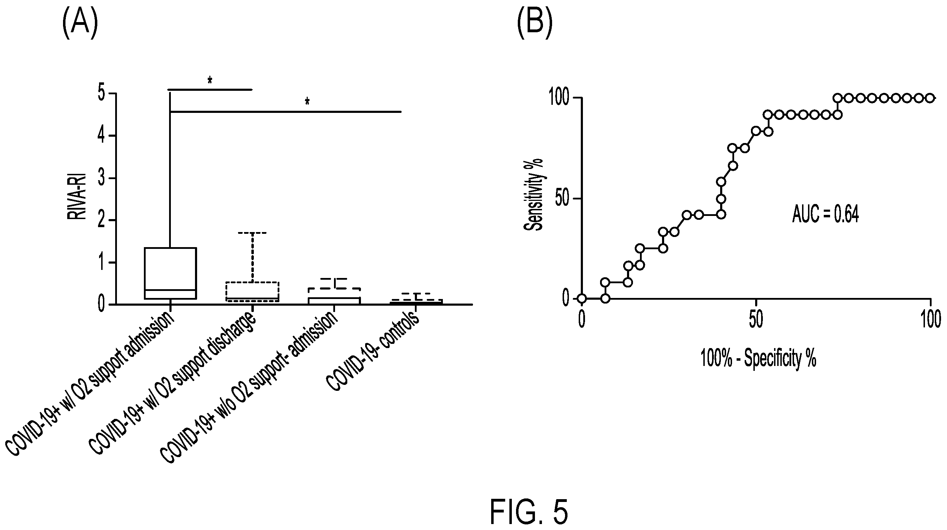

The weighted contributions of the amplitudes of the respiratory signal (f_{R0}) can be ratiometrically normalized to amplitude of the frequency of the pulse rate (f₀) to produce a Respiratory non-Invasive Venous waveform Analysis - respiratory index (RIVA-RI). Fifty COVID-19+ patients that required oxygen support therapy were compared to COVID-19+ patients that did not require oxygen support, as shown in Figure 5.

The RIVA-RI for COVID-19 positive patients (COVID-19+) admitted to the hospital and requiring oxygen support during hospitalization (median = 0.27, n = 34) was higher (p < 0.01, 95% CI 0.4008 - 2.037) than the RIVA-RI for COVID-19 negative controls (median = 0.06, n = 34). The RIVA-RI for COVID-19 + patients that required oxygen support was also higher (p = 0.02, 95% CI 0.1023 - 1.939) than the RIVA-RI for those same patients at time of discharge (median = 0.12, n = 24). The RIVA-RI was not different (p = 0.09, 95% CI -0.1242 - 2.265) for COVID-19+ patients that required oxygen support during hospitalization and those COVID-19+ patients that never required oxygen support during hospitalization (median = 0.2, n = 11). Statistical analysis was completed with multiple comparisons between groups. Horizontal bars with star (*) demonstrate statistical significance. The ability of the RIVA-RI for predicting the need for oxygen support (B) during admission for COVID-19 positive patients demonstrated an AUC of 0.64 (95% CI, 0.48- 0.81). At a RIVA-RI value of ≥ 0.6, the result was a 92% sensitivity (95% CI, 61.52% to 99.79%) and 47% specificity (95% CI, 28.34% to 65.67%) for predicting need for oxygen support during admission for COVID-19 positive patients. The above abbreviations are defined as follows: COVID 19+ = positive for the SARS-CoV-2 virus; COVID-19- = negative for the SARS-CoV-2 virus; O₂ = oxygen; RIVA-RI= Respiratory non-Invasive Venous waveform Analysis - Respiratory Index; AUC= area under the curve.

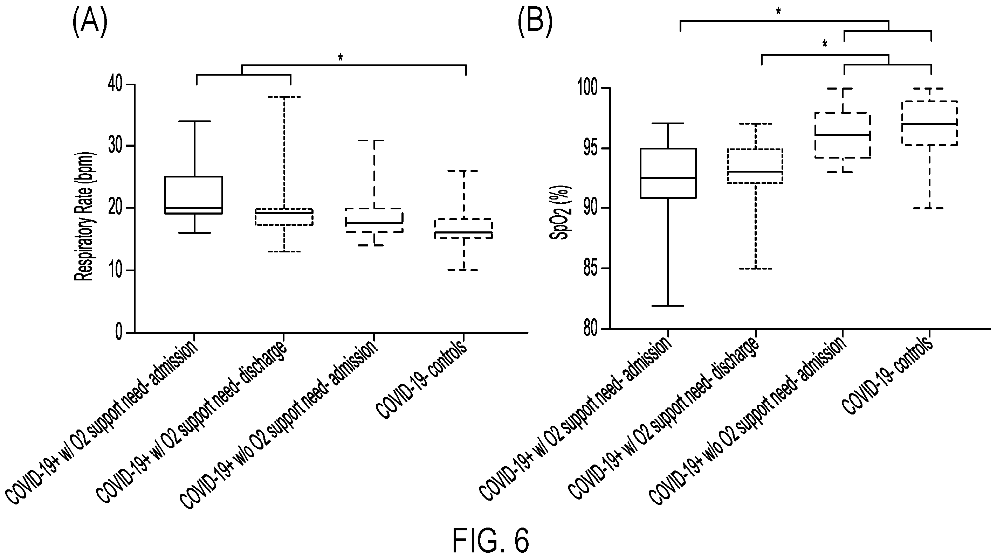

Patients that required oxygen support therapy had a significantly higher (p=0.02, 95% CI 0.1023 - 1.039) median RIVA-RI (median=0.6, n=34) compared to discharge RIVA-RI (median= 0.12, n=24) and significantly higher (p<0.01, 95% CI 0.4008-2.037) than the median RIVA-RI of healthy controls (median=0.06, n=34) (Figure 3). The ability of RIVA-RI to predict the need for oxygen support therapy during admission for COVID-19+ patients demonstrated an AUC=0.64 (95% CI, 0.48-0.81). A key point on the ROC curve demonstrated that a RIVA-RI value of ≥ 0.6 had a 92% sensitivity (95% CI, 61.52% to 99.79%) and 47% specificity (95% CI, 28.34% to 65.67%) in predicting a COVID-19+ patients' future need for oxygen support therapy. Neither respiratory rate (RR) nor oxygen saturation determined with pulse oximetry (SpO₂) demonstrated any relationship to oxygen support therapy needs in COVID-19 patients in this study, as shown in Figure 6.

There was no significant difference (p = 0.13, 95% CI -0.7309 - 8.28) in the respiratory rate (A) between COVID-19 positive patients (COVID-19+) requiring oxygen support during hospitalization (n = 34) and those without oxygen support during hospitalization (n =11) or between COVID-19 positive patients on admission to the hospital (n = 34) and at discharge (n = 27; p = 0.66, 95% CI -1.944 - 4.974). COVID-19+ patients that required oxygen support during hospitalization had a significantly lower oxygen saturation (B, SpO₂) on admission (n = 34; p < 0.01, 95% CI 2.536 - 8.727) and at discharge (n = 27; p < 0.01, 95% CI 0.8066 - 7.426) than COVID-19 positive patients that did not require oxygen support during hospitalization (n = 12). Statistical analysis was completed with multiple comparisons between groups. Horizontal bars with star (*) demonstrate statistical significance.

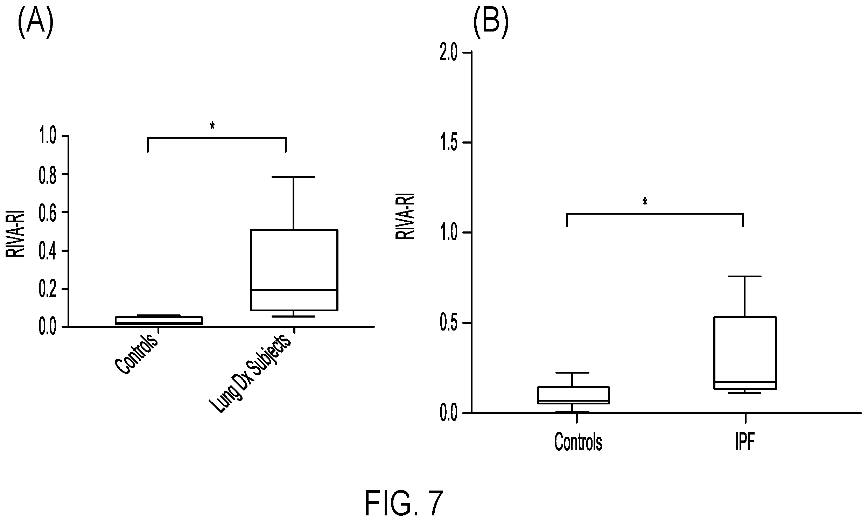

An additional proof of concept study was performed in patients with chronic pulmonary conditions that were undergoing pulmonary function tests (PFT) or had right heart catheterizations (RHC). Retrospective analysis of the respiratory component of the venous waveform from 8 patients with pulmonary disease (COPD, pulmonary fibrosis, or emphysema: "lung dx") and in 10 controls provided a RIVA-RI that was significantly (p < 0.05,) elevated in patients with lung disease, as shown in Figure 7.

Figure 7 shows a boxplot of difference in RIVA-RI between control and patients with lung disease (A) and RIV A-RI for Idiopathic Pulmonary Fibrosis (B). Venous waveforms were obtained with the NIVA device in patients undergoing right heart catheterization. Retrospective analysis of the respiratory component of the venous waveform from 8 patients with pulmonary disease (COPD, pulmonary fibrosis, or emphysema: "lung dx") and in 10 controls provided a RIVA-RI that was significantly (p < 0.05) elevated in patients with lung disease (A). When investigated in IPF, the RIVA-RI was (N=5, median=0.18, IQR 0.14-0.54) compared to pulmonary healthy controls (N=33, median=0.06, IQR 0.03-0.13). Abbreviations are defined as follows: RIVA-RI= Respiratory non-Invasive Venous waveform Analysis-respiratory index; IPF: idiopathic pulmonary fibrosis. (*)= statistically significant <0.05. There was an elevated RIVA-RI in subjects with the clinical diagnosis of lung disease and in patients with the clinical diagnosis of IPF.

RIVA-RI was investigated in patients having the clinical diagnosis of Idiopathic Pulmonary Fibrosis (N=5, IPF) and a comparison was conducted to determine the significance between IPF and health controls. Healthy control patients displayed significantly lower RIVA-RI values compared to patients with idiopathic pulmonary fibrosis (IPF; p < 0.05). There were no significant correlations between RIVA-RI and any specific value associated with the pulmonary function test (e.g., FEV1, FVC, DLCO), suggesting that RIVA-RI provides a value unique from those obtained with PFT's. Thus, elevated RIVA-RI was associated with chronic pulmonary disease and restrictive diseases (IPF) in particular.

Finally, RIVA-RI was obtained in four healthy adult patients concurrently with VO₂max. VO₂ max (mL/kg/min) refers to the intensity of aerobic (oxidative phosphorylation) process and denotes the maximum capacity of transport and utilization of oxygen during exercise at increasing intensities. It is the highest rate of oxygen consumption attainable for a particular individual. At a person's VO₂ max, any additional adenosine triphosphate (ATP) demands must come from other, non-O₂ dependent, metabolic pathways, such as the anaerobic (glycolysis) pathway and the breakdown of pyruvate. VO₂ max, in essence, mimics maximal pulmonary function. Similarly, as a patient's respiratory condition worsens secondary to a pneumonia or COVID-19+ their ability to transport and utilize oxygen worsens requiring oxygen support therapy and more reliance on compensatory metabolic pathways for ATP. When each adult (N=4, Ages= 38 years, 34 years, 22 years, and 21 years) reached their VO₂ max, RIVA-RI increased from a median baseline of 0.03 to a value of 0.78 suggesting that RIVA-RI increases relative to increasing oxygen demand.

Taken together, these data suggest that RIVA-RI measures the ability to transport and use oxygen at rest and during physical activity (or exertion, work, exercise)." Hence, RIVA-RI may be used to determine and follow trends, in the extent of functional limitation due to pulmonary disease, to assist with diagnosis and management of patients with acute and chronic COVID-19 disease.

Acquisition, processing, and analysis of the venous waveform using a piezoelectric sensor on the volar aspect of the wrist, uses a unique physiologic signal, the venous waveforms (not simply venous pressure measurements) for monitoring. Deconvolution of a series of waveforms ("window of data over time"), with an algorithm (fast Fourier transformation), demonstrates the presence of a respiratory signal derived from waves created by retrograde transmission of the negative pressure exerted on the venous system by inspiratory pressures. Weighted contributions of the amplitudes of the frequencies of the pulse rate (f₀) and the respiratory signal (f_{R0}) were used to create a respiratory index ("RIVA-RI"). Preliminary data generated using RIVA-RI suggest that the characteristics of the respiratory signal in the peripheral venous waveform could be used to provide the first point of care, non-invasive device for physiologic monitoring and risk stratification of respiratory disease, including patients with COVID-19.

Current clinical decision making used to determine the need for hospitalization and/or level of respiratory support in COVID-19 patients is based on respiratory rate (RR, a vital sign), SpO₂ (peripheral capillary oxygen saturation, obtained by pulse oximetry), and clinical judgement. While RIVA-RI had predictive value for predicting the need for supplemental oxygen therapy, both RR and SpO₂ failed to detect need for oxygen therapy (see Figure 6) in COVID-19 patients. The introduction of pulse oximetry was a major advancement in medical monitoring, however there remain problems in SpO₂ demonstrating a lack of specificity in monitoring for acute disease. Respiratory rate has been found to be a very important vital sign for predicting clinical outcomes; however, time and time again its limitations of usage and inaccurate measurements are described. Thus, RIVA-RI value would be the first approach to provide quantitative risk stratification of respiratory physiology, clinically meaningful information to monitor acute respiratory failure, and help triage the need for oxygen therapy in patients with respiratory distress.

In reviewing venous waveforms in patients with chronic lung disease, elevated RIVA-RI is associated with restrictive chronic pulmonary disease (IPF, see Figure 7). COVID-19 and/or prolonged ventilator use associated with COVID-19 can lead to chronic pulmonary fibrosis and chronic impairment of pulmonary function (one aspect of "long COVID"), suggesting that RIVA-RI may be useful in long term follow up of COVID-19 patients. These preliminary data suggest that RIVA-RI could be used across the spectrum of severe respiratory diseases such as COVID-19.

With careful clinical analyses, it is likely that RIVA-RI would have additional future uses in monitoring other acute and chronic respiratory diseases such pulmonary fibrosis at home, in the clinic, and in hospital settings. RIVA-RI will likely have further potential to monitor respiratory status in hospitalized patients especially in determining the level of oxygen support needed (oxygen, CPAP, ventilator) and assist in determining how to appropriately decrease that level of oxygen support as respiratory status improves. The ability to use RIVA-RI in home settings, and the additional monitoring potential that a wristband device with a photodiode and piezoelectric sensor could provide (RR, SpO₂, heart rate, and volume status), suggest that RIVA-RI could be a primary physiologic monitor for telehealth applications in patients with COVID-19, and a variety of other respiratory diseases in the future. The ease of use (disposable wrist patch or reusable wristband), point of care availability, non-invasiveness, and accuracy, position RIVA-RI as a unique and innovative respiratory monitor.

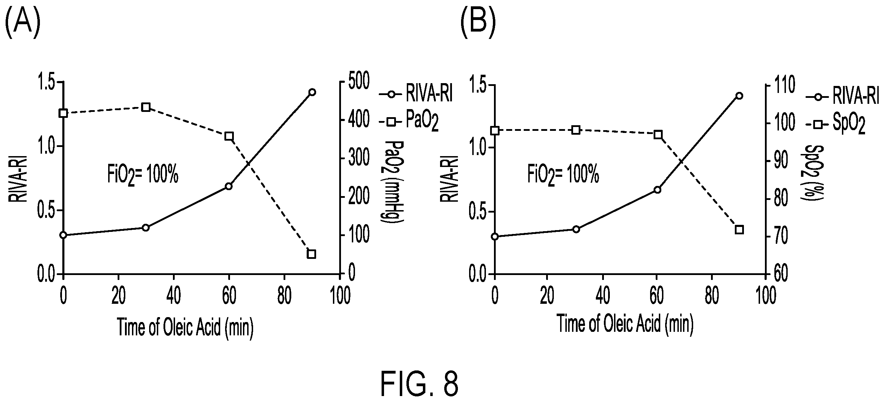

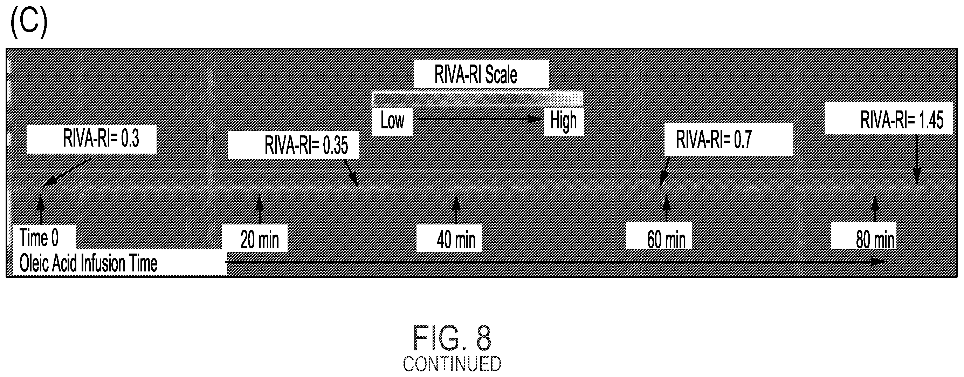

Figure 8 shows relationships between RIVA-RI to PaO₂ and SpO₂ over time of oleic acid infusion in a porcine model. As acute respiratory distress is induced through direct infusion of oleic acid into the pulmonary artery, RIVA-RI increases as PaO₂ (A) and SpO₂ (B) decreases. When RIVA-RI is visualized in the color spectrum over the entire course of oleic acid infusion, an increase in RIVA-RI value can be seen after 40 minutes of infusion and increasing throughout the development of respiratory distress. Abbreviations are defined as follows: RIVA-RI= Respiratory non-Invasive Venous waveform Analysis Respiratory Index, PaO₂= partial pressure of oxygen, SpO₂= oxygen hemoglobin saturation, min= minutes.

All the above practices have been successfully demonstrated in the data shown in Figure 8. The model has demonstrated increases in RIVA-RI prior to changes in other respiratory monitoring measures (RR and SpO₂) in patients with COVID. Data also showed that RIVA-RI increases occurred before decreases in PaO₂ and well before decreases in SpO₂ in the porcine model. The ability is confirmed of RIVA-RI to predict the need for subsequent therapeutic oxygen support and determine the RIVA-RI response to ARDS/COVID-19 treatment strategies.

## Claims

1. A method (300) comprising:
generating (302), via a sensor (114), a signal representing vibrations originating from a blood vessel of a patient;
generating (304) an intensity spectrum of the signal that indicates intensities of the vibrations with respect to oscillation frequencies of the vibrations;
identifying (306) a first peak of the intensity spectrum that corresponds to a respiratory frequency of the patient and a second peak of the intensity spectrum that corresponds to a heart rate of the patient;
performing a comparison (308) of a first intensity of the first peak with a second intensity of the second peak; and
generating (310), via a user interface (112), output indicative of the comparison.

2. The method of claim 1, further comprising pressing the sensor (114) to a skin of the patient while generating the signal.

3. The method of any of claims 1-2, wherein the blood vessel comprises a peripheral vein.

4. The method of any of claims 1-3, wherein identifying (306) the first peak comprises identifying the first peak as being a most intense peak of any peaks within a range of 0.1 Hz to 0.5 Hz.

5. The method of any of claims 1-4, wherein performing the comparison (308) comprises calculating a ratio of the first intensity to the second intensity or a ratio of the second intensity to the first intensity.

6. The method of any of claims 1-5, further comprising determining, based on the comparison (308), a diffusing capacity of the patient's lung for carbon monoxide (DLCO), wherein generating (310) the output comprises generating the output such that the output indicates the DLCO.

7. The method of any of claims 1-6, further comprising determining based on the comparison (308), a diffusing capacity of the patient's lung for carbon monoxide divided by an alveolar volume (DLCO/VA), wherein generating (310) the output comprises generating the output such that the output indicates the DLCO/VA.

8. The method of any of claims 1-7, further comprising determining, based on the comparison (308), an inspiratory reserve volume (IRV) for the patient's lung, wherein generating (310) the output comprises generating the output such that the output indicates the IRV.

9. The method of any of claims 1-8, further comprising determining, based on the comparison (308), an inspiratory capacity (IC) for the patient's lung, wherein generating (310) the output comprises generating the output such that the output indicates the IC.

10. The method of any of claims 1-9, further comprising determining, based on the comparison (308), a maximum rate of oxygen consumption (VO₂ max), wherein generating (310) the output comprises generating the output such that the output indicates the VO₂ max.

11. The method of any of claims 1-10, further comprising determining, based on the comparison (308), a work of breathing for the patient, wherein generating (310) the output comprises generating the output such that the output indicates the work of breathing.

12. The method of claims of 1-11, further comprising determining, based on the comparison (308), a pleural pressure for the patient's lung, wherein generating (310) the output comprises generating the output such that the output indicates the pleural pressure.

13. A system (100) comprising:
one or more processors (102);
a user interface (112);
a sensor (114); and
a computer readable medium (104) storing instructions (106) that, when executed by the one or more processors (102), cause the system (100) to perform the method of any one of claims 1-12.

14. A non-transitory computer readable medium (104) storing instructions (106) that, when executed by the one or more processors (102) of the system (100) of claim 13, cause the system (100) to perform the method of any of claims 1-12.

## Patentansprüche

1. Verfahren (300), umfassend:
Erzeugen (302) eines Signals über einen Sensor (114), das von einem Blutgefäß eines Patienten ausgehende Schwingungen darstellt;
Erzeugen (304) eines Intensitätsspektrums des Signals, das die Intensitäten der Schwingungen in Abhängigkeit von den Schwingungsfrequenzen der Schwingungen angibt;
Identifizieren (306) eines ersten Peaks des Intensitätsspektrums, der einer Atemfrequenz des Patienten entspricht, und eines zweiten Peaks des Intensitätsspektrums, der einer Herzfrequenz des Patienten entspricht;
Durchführen eines Vergleichs (308) einer ersten Intensität des ersten Peaks mit einer zweiten Intensität des zweiten Peaks; und
Erzeugen (310) einer Ausgabe, die den Vergleich anzeigt, über eine Benutzerschnittstelle (112).

2. Verfahren nach Anspruch 1, das ferner das Andrücken des Sensors (114) an die Haut des Patienten während der Erzeugung des Signals umfasst.

3. Verfahren nach einem der Ansprüche 1 bis 2, wobei das Blutgefäß eine periphere Vene umfasst.

4. Verfahren nach einem der Ansprüche 1 bis 3, wobei das Identifizieren (306) der ersten Spitze das Identifizieren der ersten Spitze als die intensivste Spitze aller Spitzen innerhalb eines Bereichs von 0,1 Hz bis 0,5 Hz umfasst.

5. Verfahren nach einem der Ansprüche 1 bis 4, wobei das Durchführen des Vergleichs (308) das Berechnen eines Verhältnisses der ersten Intensität zur zweiten Intensität oder eines Verhältnisses der zweiten Intensität zur ersten Intensität umfasst.

6. Verfahren nach einem der Ansprüche 1 bis 5, das ferner das Bestimmen einer Diffusionskapazität der Lunge des Patienten für Kohlenmonoxid (DLCO) auf der Grundlage des Vergleichs (308) umfasst, wobei das Erzeugen (310) der Ausgabe das Erzeugen der Ausgabe derart umfasst, dass die Ausgabe die DLCO angibt.

7. Verfahren nach einem der Ansprüche 1 bis 6, das ferner das Bestimmen einer Diffusionskapazität der Lunge des Patienten für Kohlenmonoxid, geteilt durch ein Alveolarvolumen (DLCO/VA), auf der Grundlage des Vergleichs (308) umfasst, wobei das Erzeugen (310) der Ausgabe das Erzeugen der Ausgabe in einer Weise umfasst, dass die Ausgabe den DLCO/VA angibt.

8. Verfahren nach einem der Ansprüche 1 bis 7, das ferner das Bestimmen eines inspiratorischen Reservevolumens (IRV) für die Lunge des Patienten auf der Grundlage des Vergleichs (308) umfasst, wobei das Erzeugen (310) des Ausgangssignals das Erzeugen des Ausgangssignals in einer Weise umfasst, dass das Ausgangssignal das IRV angibt.

9. Verfahren nach einem der Ansprüche 1 bis 8, das ferner das Bestimmen einer Inspirationskapazität (IC) für die Lunge des Patienten auf der Grundlage des Vergleichs (308) umfasst, wobei das Erzeugen (310) des Ausgangssignals das Erzeugen des Ausgangssignals in einer Weise umfasst, dass das Ausgangssignal die IC angibt.

10. Verfahren nach einem der Ansprüche 1 bis 9, das ferner das Bestimmen einer maximalen Sauerstoffverbrauchsrate (VO₂ max) auf der Grundlage des Vergleichs (308) umfasst, wobei das Erzeugen (310) des Ausgangssignals das Erzeugen des Ausgangssignals in einer Weise umfasst, dass das Ausgangssignal die VO₂ max angibt.

11. Verfahren nach einem der Ansprüche 1 bis 10, das ferner das Bestimmen einer Atemarbeit für den Patienten auf der Grundlage des Vergleichs (308) umfasst, wobei das Erzeugen (310) des Ausgangssignals das Erzeugen des Ausgangssignals in einer Weise umfasst, dass das Ausgangssignal die Atemarbeit angibt.

12. Verfahren nach einem der Ansprüche 1 bis 11, das ferner das Bestimmen eines Pleuradrucks für die Lunge des Patienten auf der Grundlage des Vergleichs (308) umfasst, wobei das Erzeugen (310) der Ausgabe das Erzeugen der Ausgabe in einer Weise umfasst, dass die Ausgabe den Pleuradruck angibt.

13. System (100), das umfasst:
einen oder mehrere Prozessoren (102);
eine Benutzerschnittstelle (112);
einen Sensor (114); und
ein computerlesbares Medium (104), das Anweisungen (106) speichert, die, wenn sie von dem einen oder den mehreren Prozessoren (102) ausgeführt werden, bewirken, dass das System (100) das Verfahren nach einem der Ansprüche 1 bis 12 ausführt.

14. Ein nicht-flüchtiges, computerlesbares Medium (104), das Befehle (106) speichert, die, wenn sie von dem einen oder den mehreren Prozessoren (102) des Systems (100) nach Anspruch 13 ausgeführt werden, bewirken, dass das System (100) das Verfahren nach einem der Ansprüche 1 bis 12 ausführt.

## Revendications

1. Procédé (300) comprenant :
la génération (302), via un capteur (114), d'un signal représentant des vibrations provenant d'un vaisseau sanguin d'un patient ;
la génération (304) d'un spectre d'intensité du signal qui indique les intensités des vibrations en fonction des fréquences d'oscillation de ces vibrations ;
l'identification (306) d'un premier pic du spectre d'intensité qui correspond à une fréquence respiratoire du patient et d'un deuxième pic du spectre d'intensité qui correspond à une fréquence cardiaque du patient ;
la réalisation d'une comparaison (308) entre une première intensité du premier pic et une deuxième intensité du deuxième pic ; et
la génération (310), via une interface utilisateur (112), d'une sortie indicative de la comparaison.

2. Procédé selon la revendication 1, comprenant en outre l'application du capteur (114) sur la peau du patient tout en générant le signal.

3. Procédé selon l'une quelconque des revendications 1 à 2, dans lequel le vaisseau sanguin comprend une veine périphérique.

4. Procédé selon l'une quelconque des revendications 1 à 3, dans lequel l'identification (306) du premier pic comprend l'identification du premier pic comme étant le pic le plus intense parmi tous les pics compris dans une plage de 0,1 Hz à 0,5 Hz.

5. Procédé selon l'une quelconque des revendications 1 à 4, dans lequel la réalisation de la comparaison (308) comprend le calcul d'un rapport entre la première intensité et la deuxième intensité ou d'un rapport entre la deuxième intensité et la première intensité.

6. Procédé selon l'une quelconque des revendications 1 à 5, comprenant en outre la détermination, sur la base de la comparaison (308), d'une capacité de diffusion du monoxyde de carbone dans les poumons du patient (DLCO), dans lequel la génération (310) de la sortie comprend la génération de la sortie de telle sorte que celle-ci indique la DLCO.

7. Procédé selon l'une quelconque des revendications 1 à 6, comprenant en outre la détermination, sur la base de la comparaison (308), d'une capacité de diffusion du monoxyde de carbone dans les poumons du patient divisée par un volume alvéolaire (DLCO/VA), dans lequel la génération (310) de la sortie comprend la génération de la sortie de telle sorte que celle-ci indique la DLCO/VA.

8. Procédé selon l'une quelconque des revendications 1 à 7, comprenant en outre la détermination, sur la base de la comparaison (308), d'un volume de réserve inspiratoire (IRV) pour le poumon du patient, dans lequel la génération (310) de la sortie comprend la génération de la sortie de telle sorte que celle-ci indique l'IRV.

9. Procédé selon l'une quelconque des revendications 1 à 8, comprenant en outre la détermination, sur la base de la comparaison (308), d'une capacité inspiratoire (IC) pour le poumon du patient, dans lequel la génération (310) de la sortie comprend la génération de la sortie de telle sorte que celle-ci indique l'IC.

10. Procédé selon l'une quelconque des revendications 1 à 9, comprenant en outre la détermination, sur la base de la comparaison (308), d'un débit maximal de consommation d'oxygène (VO₂ max), dans lequel la génération (310) de la sortie comprend la génération de la sortie de telle sorte que celle-ci indique le VO₂ max.

11. Procédé selon l'une quelconque des revendications 1 à 10, comprenant en outre la détermination, sur la base de la comparaison (308), d'un travail respiratoire pour le patient, dans lequel la génération (310) de la sortie comprend la génération de la sortie de telle sorte que celle-ci indique le travail respiratoire.

12. Procédé selon l'une quelconque des revendications 1 à 11, comprenant en outre la détermination, sur la base de la comparaison (308), d'une pression pleurale pour le poumon du patient, dans lequel la génération (310) de la sortie comprend la génération de la sortie de telle sorte que celle-ci indique la pression pleurale.

13. Système (100) comprenant :
un ou plusieurs processeurs (102) ;
une interface utilisateur (112) ;
un capteur (114) ; et
un support lisible par ordinateur (104) stockant des instructions (106) qui, lorsqu'elles sont exécutées par le ou les processeurs (102), amènent le système (100) à mettre en œuvre le procédé selon l'une quelconque des revendications 1 à 12.

14. Un support lisible par ordinateur non transitoire (104) stockant des instructions (106) qui, lorsqu'elles sont exécutées par le ou les processeurs (102) du système (100) de la revendication 13, amènent le système (100) à mettre en œuvre le procédé de l'une quelconque des revendications 1 à 12.
